# EUROPÄISCHE PATENTSCHRIFT

(11) **EP 0 810 822 B1**
(45) Veröffentlichungstag und Bekanntmachung des Hinweises auf die Patenterteilung: **22.05.2002**
(21) Anmeldenummer: 96902991.7
(22) Anmeldetag: 22.02.1996
(51) Int. Cl.: A01N 25/08

(54) **VERWENDUNG VON AEROGELEN IN DER LANDWIRTSCHAFT**
USE OF AEROGELS IN AGRICULTURE
UTILISATION D'AEROGELS EN AGRICULTURE

(30) Priorität: 22.02.1995 DE 19506141
(43) Veröffentlichungstag der Anmeldung: 10.12.1997
(73) Patentinhaber: CABOT CORPORATION, Boston, Massachusetts 02210-2019 (US)
(72) Erfinder: FRISCH, Gerhard, D-61273 Wehrheim (DE); ZIMMERMANN, Andreas, D-64347 Griesheim (DE); SCHWERTFEGER, Fritz, D-60529 Frankfurt am Main (DE)
(74) Vertreter: Luderschmidt, Schüler & Partner GbR
(86) Internationale Anmeldenummer: EP9600725
(87) Internationale Veröffentlichungsnummer: WO9625850

(56) Entgegenhaltungen:
- EP-A- 0 171 722
- WO-A-94/25149
- DE-A- 2 652 163
- US-A- 3 235 451
- DATABASE WPI Section Ch, Week 9411 Derwent Publications Ltd., London, GB; Class B07, AN 94-089171 XP002007255 & JP,A,06 040 714 (SHIONOGI & CO LTD) , 15.Februar 1994
- CHEMICAL ABSTRACTS, vol. 69, no. 23, 2.Dezember 1968 Columbus, Ohio, US; abstract no. 95399, W. EBELING, D.A. REIERSON & R.E. WAGNER: "Influence of repellency on the efficacy of blatticides.IV.Comparison of four cockroach species." Seite 8913; XP002007253 & J. ECON. ENTOMOL., Bd. 61, Nr. 5, 1968, Seiten 1213-1219,
- CHEMICAL ABSTRACTS, vol. 92, no. 12, 24.März 1980 Columbus, Ohio, US; abstract no. 99465q, S. SUZUKI & T. HIDAKA: "Gelled solid containing volatile active ingredients" Seite 337; XP002007254 & JP,A,54 092 630 (JAPAN SYNTHETIC RUBBER) 23.Juli 1979

## Beschreibung

Die Erfindung betrifft die Verwendung von Aerogelen z.B. im landwirtschaftlichen und veterinärmedizischen Bereich als Trägermaterial für aktiv wirksame Substanzen.

Aerogele, insbesondere solche mit Porositäten über 60 % und Dichten unter 0,6 g/cm³, weisen eine äußerst geringe thermische Leitfähigkeit auf und finden deshalb Anwendung als Wärmeisolationsmaterial wie z.B. in der EP-A-0 171 722 beschrieben.

Aerogele im weiteren Sinn, d.h. im Sinne von "Gel mit Luft als Dispersionsmittel", werden durch Trocknung eines geeigneten Gels hergestellt. Unter den Begriff "Aerogel" in diesem Sinne, fallen Aerogele im engeren Sinn, Xerogele und Kryogele. Dabei wird ein getrocknetes Gel als Aerogel im engeren Sinn bezeichnet, wenn die Flüssigkeit des Gels bei Temperaturen oberhalb der kritischen Temperatur und ausgehend von Drücken oberhalb des kritischen Druckes weitestgehend entfernt wird. Wird die Flüssigkeit des Gels dagegen unterkritisch, beispielsweise unter Bildung einer Flüssig-Dampf-Grenzphase entfernt, dann bezeichnet man das entstandene Gel als Xerogel.

Bei der Verwendung des Begriffs Aerogele in der vorliegenden Anmeldung handelt es sich um Aerogele im weiteren Sinn, d.h. im Sinn von "Gel mit Luft als Dispersionsmittel".

Darüber hinaus kann man die Aerogele grundsätzlich in anorganische und organische Aerogele unterteilen.
Anorganische Aerogele sind schon seit 1931 bekannt (S.S. Kistler, Nature 1931, 127, 741). Seitdem sind aus unterschiedlichsten Ausgangsmaterialien Aerogele dargestellt worden. Dabei konnten z.B. SiO₂-, Al₂O₃-, TiO₂-, ZrO₂-, SnO₂-, Li₂O-, CeO₂-, V₂O₅-Aerogele und Mischungen aus diesen hergestellt werden (H.D. Gesser, P.C. Goswami, Chem. Rev. 1989, 89, 756ff).
Seit einigen Jahren sind auch organische Aerogele aus unterschiedlichsten Ausgangsmaterialien, wie z.B. aus Melaminformaldehyd, bekannt (R.W. Pekala, J. Mater, Sci. 1989, 24, 3221).

Anorganische Aerogele können dabei auf unterschiedlichsten Wegen hergestellt werden.

Beispielsweise können SiO₂-Aerogele durch saure Hydrolyse und Kondensation von Tetraethylorthosilikat in Ethanol hergestellt werden. Dabei entsteht ein Gel, das durch überkritische Trocknung unter Erhaltung der Struktur getrocknet werden kann. Auf dieser Trocknungstechnik basierende Herstellungsverfahren sind z.B. aus der EP-A-0 396 076 oder der WO 92/03378 bekannt.

Eine Alternative bietet ein Verfahren zur unterkritischen Trocknung von SiO₂-Gelen, wenn diese vor der Trocknung mit einem chlorhaltigen Silylierungsmittel umgesetzt werden. Das SiO₂-Gel kann dabei beispielsweise durch saure Hydrolyse von Tetraalkoxysilanen in einem geeigneten organischen Lösungsmittel mittels Wasser erhalten werden. Nach Austausch des Lösungsmittels gegen ein geeignetes organisches Lösungsmittel wird in einem weiteren Schritt das erhaltene Gel mit einem Silylierungsmittel umgesetzt. Das dabei entstehende SiO₂-Gel kann anschließend aus einem organischen Lösungsmittel heraus an der Luft getrocknet werden. Damit können Aerogele mit Dichten unter 0,4 g/cm³ und Porositäten über 60 % erreicht werden.

Das auf dieser Trocknungstechnik basierende Herstellungsverfahren ist ausführlich in der WO 94/25149 beschrieben.

Die oben beschriebenen Gele können darüber hinaus vor der Trocknung in der alkohol-wäßrigen Lösung mit Tetraalkoxysilanen versetzt und gealtert werden, um die Gelnetzwerkstärke zu erhöhen, wie z.B. in der WO 92/20623 offenbart.

Ferner kann das SiO₂-Gel auch auf Basis von Wasserglas hergestellt werden. Das auf dieser Technik basierende Herstellungsverfahren ist aus der DE-A-43 42 548 bekannt.

In der deutschen Patentanmeldung 19502453.2 wird darüber hinaus die Verwendung von chlorfreien Silylierungsmitteln beschrieben.

Die durch überkritische Trocknung erhaltenen Aerogele sind, je nach dem speziell angewendeten Verfahren hydrophil oder kurzfristig hydrophob. Langfristig sind sie jedoch hydrophil.

Diese Hydrophilie kann durch einen Hydrophobisierungsschritt während der überkritischen Trocknung vermieden werden. Ein solches Verfahren ist aus der EP-A-0 396 076 bekannt.

Unterkritisch getrocknete Aerogele sind bedingt durch ihr Herstellungsverfahren (Silylierung vor der Trocknung) dauerhaft hydrophob.

Aufgabe der vorliegenden Erfindung war es, nach neuen Anwendungen für Aerogele zu suchen.

Es wurde nun überraschend gefunden, daß durch Silylierung oberflächen modifizierte Aerogele im landwirtschaftlichen und veterinärmedizinischen Bereich z.B. als Trägermaterial für aktiv wirksame Substanzen geeignet sind.

Diese Wirkstoffe können aus den Bereichen der Insekticide, Fungicide, Herbicide, Acaricide, Piscicide, Rhodenticide, Molluscicide, Nematicide, Baktericide und/oder Parasiticide kommen. Ebenso können die Aerogele als Trägermaterial für Viren, Bakterien und/oder Bazillen wie z.B. Bacillus Thuringensis für die biologische Bekämpfung von nicht erwünschten Organismen dienen.

Die Wirkstoffe können entweder in gelöster und/oder in einem flüssigem Trägermedium in suspendierter Form einzeln oder zu mehreren auf dieddurch Silylierung oberflächenmodifizierten Aerogele aufgetragen oder absorbiert werden, wobei ein quasi flüssiger Zustand in den beschriebenen Hohlräumen; der Aerogele erhalten bleibt. Flüssige Wirkstoffe können auch ohne zusätzliche Trägermedien aufgenommen werden. Hierbei können diese flüssigen Mittel zusätzlich noch mit Emulgatoren ionischer wie nichtionischer Art versehen werden. Ebenso können den Aerogelen vorzugsweise nach der Aufnahme der Wirkstoffzubereitungen Netz- und Dispergiermittel zugesetzt werden. Die Größe der Aerogelpartikel ist vorzugsweise größer als 0,1 µm, besonders bevorzugt größer als 1 µm und insbesondere größer als 5 µm.

Die beladenen Aerogele können mit mindestens einem weiteren Trägermedium wie z.B. Talkum, Kreide, Kaolin und/oder vorzugsweise mit Wasser und/oder Ölen gemischt bzw. verdünnt beispielsweise auf Pflanzen, Tiere, Äcker, Landund Wasserflächen ausgebracht werden.

Bevorzugt werden anorganische Aerogele verwendet, die durch Silylierung modifiziert wurden. Unter einem anorganischen Aerogel ist in der vorliegenden Anmeldung ein Aerogel zu verstehen, das auf Basis von anorganischen Materialien hergestellt wurde.

Bevorzugt sind durch Silylierung oberflächen modifizierte Aerogele überwiegend aus SiO₂, Al₂O₃, TiO₂, ZrO₂ oder Mischungen davon. Diese weisen hydrophobe Oberflächengruppen (z.B. OH, OR, R) auf. Die Herstellung von Aerogelen mit hydrophoben Oberflächengruppen kann dabei nach allen dem Fachmann bekannten Verfahren durchgeführt werden. Besonders bevorzugt sind hydrophobe SiO₂-haltige Aerogele, insbesondere SiO₂-Aerogele.

Darüber hinaus wurde überraschend gefunden, daß die Wahl eines geeigneten hydrophoben durch Silylierung oberflächenmodifizierten Aerogels entsprechende Stoffe, mit denen das Aerogel beladen wurde, beschleunigt bzw. verzögert frei gesetzt werden können. Weiterhin können Aerogele als Dispergierungsmittel für Dispersionen von festen, flüssigen oder gasförmigen Stoffen in feste oder flüssige Medien eingesetzt werden. Darüber hinaus können mit hydrophilen und/oder hydrophoben Stoffen beladene hydrophobe Aerogele problemlos in hydrophile und/oder hydrophobe, flüssige, halbfeste bzw. feste Medien eingearbeitet werden, insbesondere, um mit Hilfe von hydrophoben Aerogelen hydrophile Stoffe in flüssige, hydrophobe Disperionsmedien einzutragen. Hydrophobe Aerogele beispielsweise, schwimmen auf hydrophilen, wäßrigen Medien auf. Ferner können auch flüssige, hydrophile oder hydrophobe Stoffe in feste, frei rieselfähige Pulver oder Granulate überführt werden.

Die Erfindung wird im folgenden anhand von Ausführungsbeispielen belegt, ohne dadurch beschränkt zu werden.

Es wird die Herstellung eines hydrophoben Aerogels beschrieben.
Dieses Aerogel wurde jeweils in den nachfolgenden Beispielen 1 bis 39 (Tabelle 1 bis 5) eingesetzt.

Die Angabe der einzelnen Bestandteile in den Tabellen sind Angaben in Gew.-% bezogen auf die Gesamtzusammensetzung.

### Herstellungsbeispiel

### Beispiel 1

### Darstellung eines dauerhaft hydrophoben Aerogels

1 I einer Natriumwasserglaslösung (mit einem Gehalt von 7 Gew.-% SiO₂ und einem Na₂O:SiO₂ Verhältnis von 1:3,3) wurde zusammen mit 0,5 I eines sauren lonenaustauscherharzes (Styroldivinylbenzolcopolymer mit Sulfonsäuregruppen, handelsüblich unter dem Namen ®Duolite C20) gerührt, bis der pH-Wert der wäßrigen Lösung 2,3 war. Anschließend wurde das lonenaustauscherharz abfiltriert und die wäßrige Lösung mit 1 molarer NaOH-Lösung auf einen pH-Wert von 5,0 eingestellt. Danach wurde das entstandene Gel noch 3 Stunden bei 85°C gealtert und anschließend das Wasser mit 3 I Aceton gegen Aceton ausgetauscht. Anschließend wurde das acetonhaltige Gel mit Trimethylchlorsilan silyliert (5 Gew.-% Trimethylchlorsilan pro Gramm nasses Gel). Die Trocknung des Gels erfolgte an Luft (3 Stunden bei 40°C, dann 2 Stunden bei 50°C und 12 Stunden bei 150°C).

Das so erhaltene, transparente Aerogel hatte eine Dichte von 0,15 g/cm³, seine spezifische Oberfläche nach BET lag bei 480 m²/g und es war dauerhaft hydrophob.

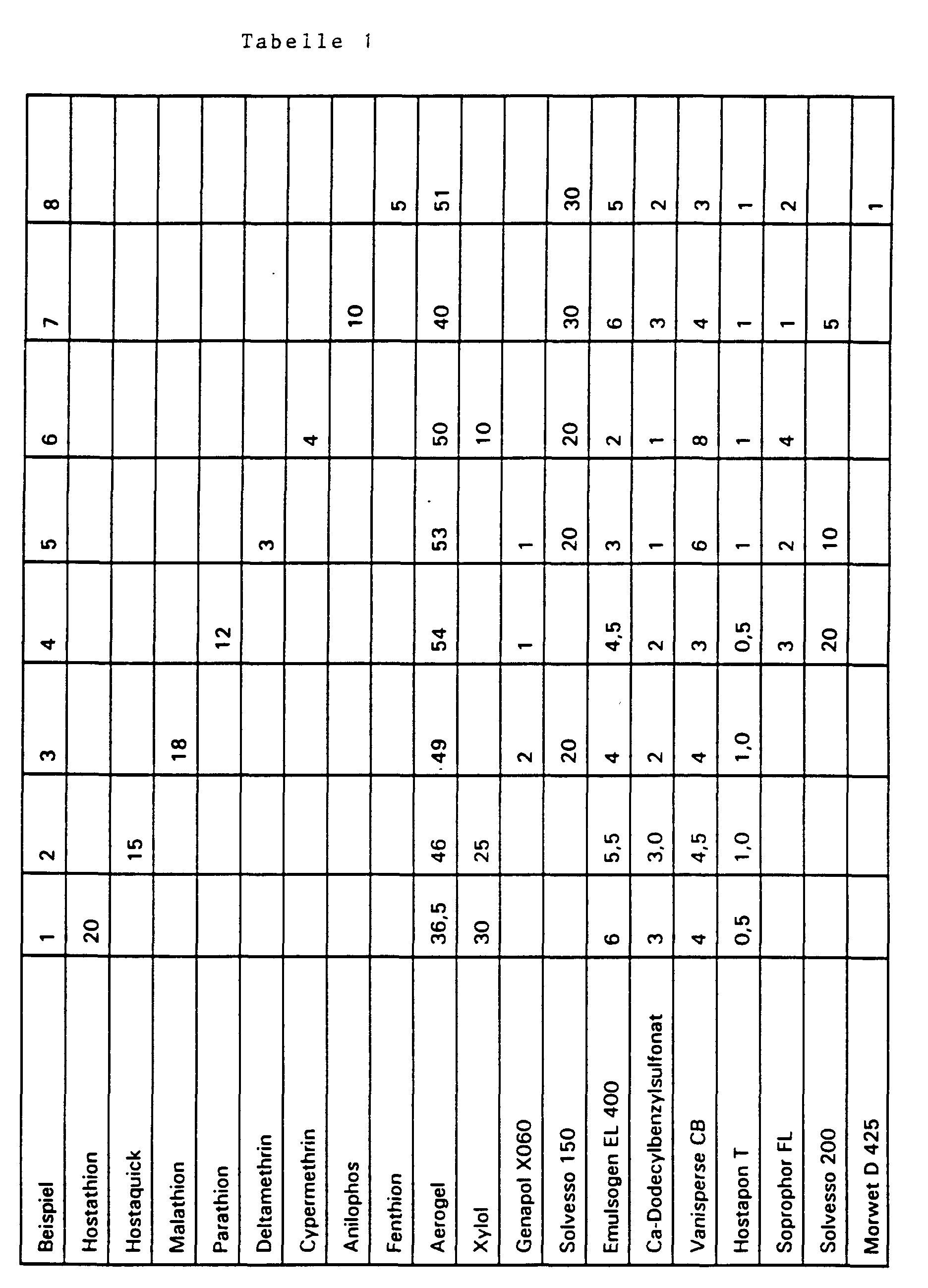

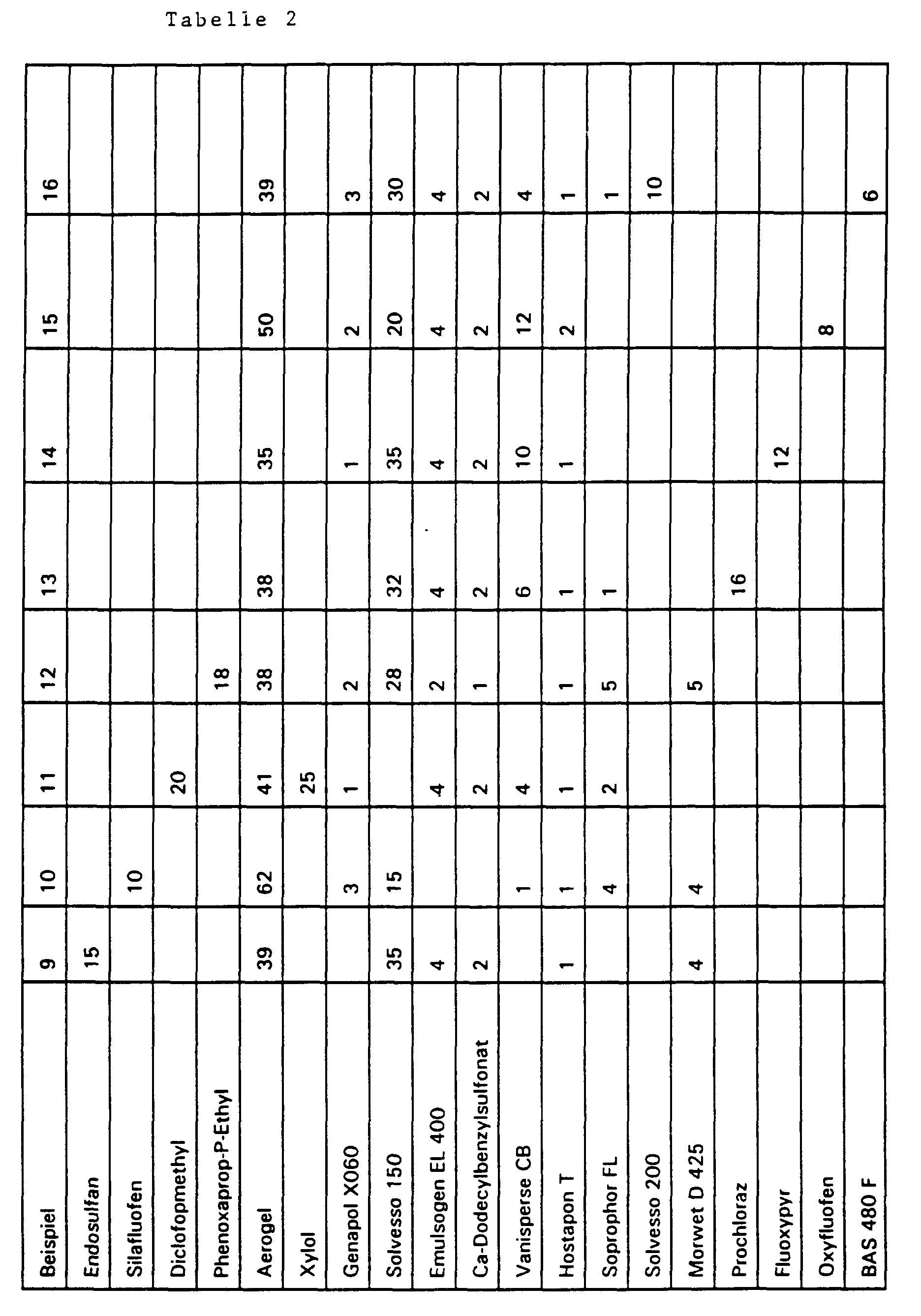

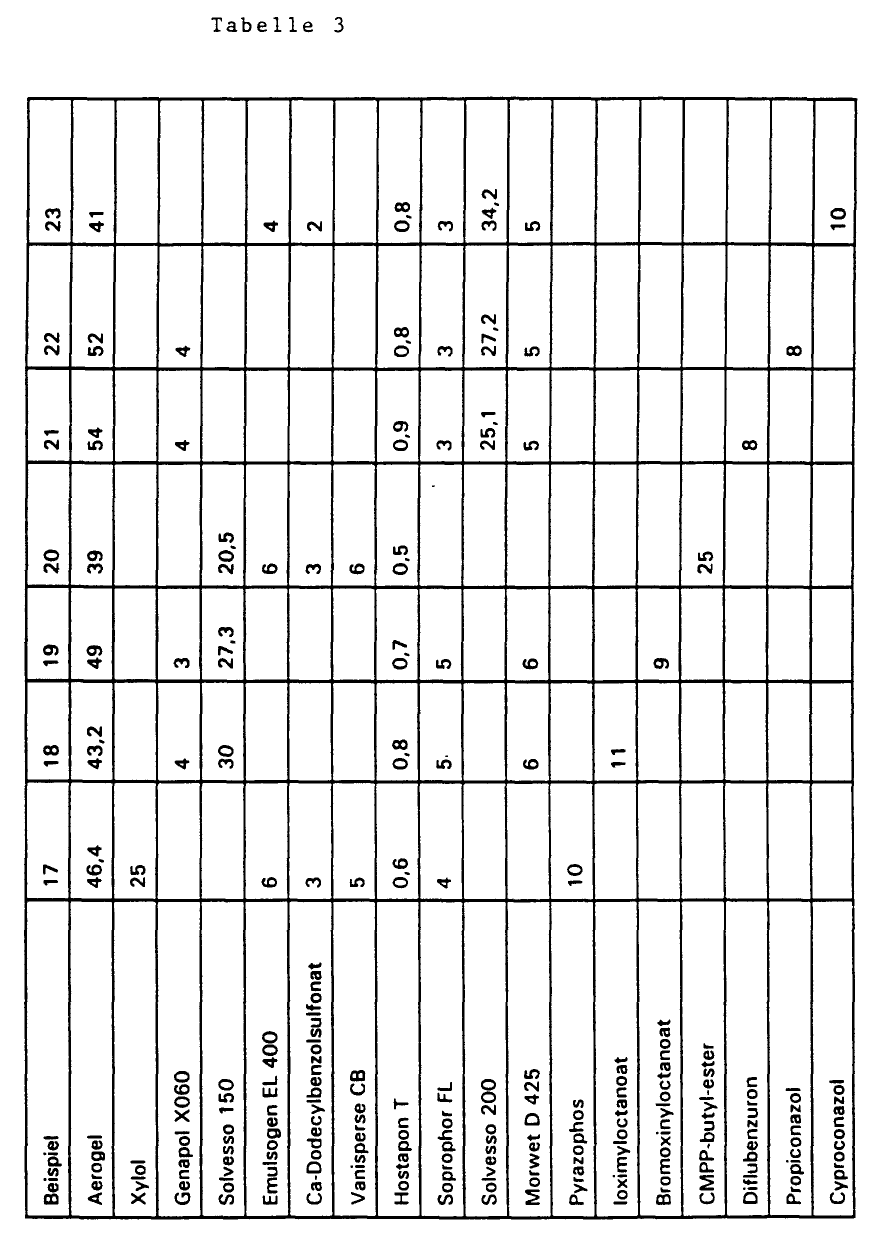

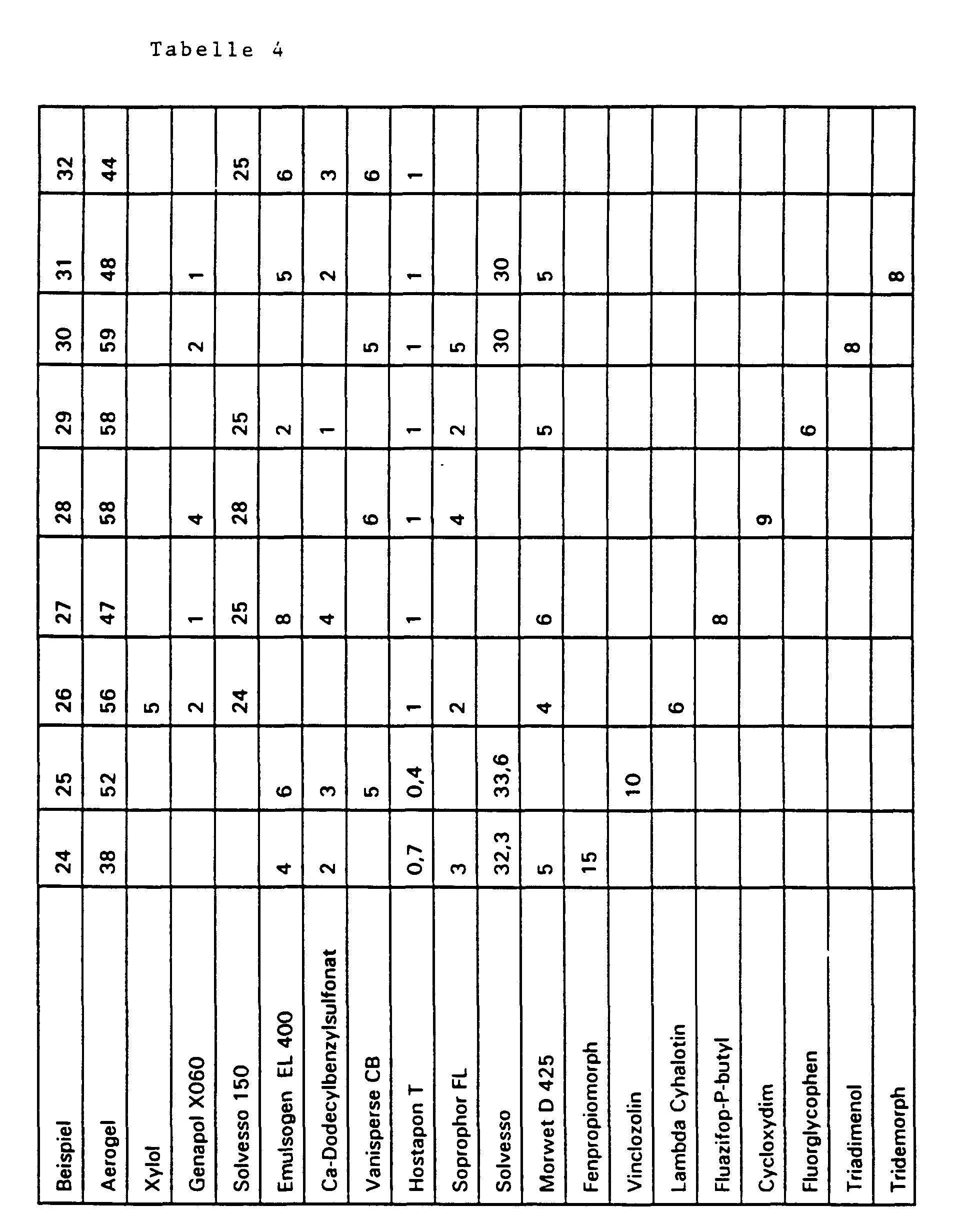

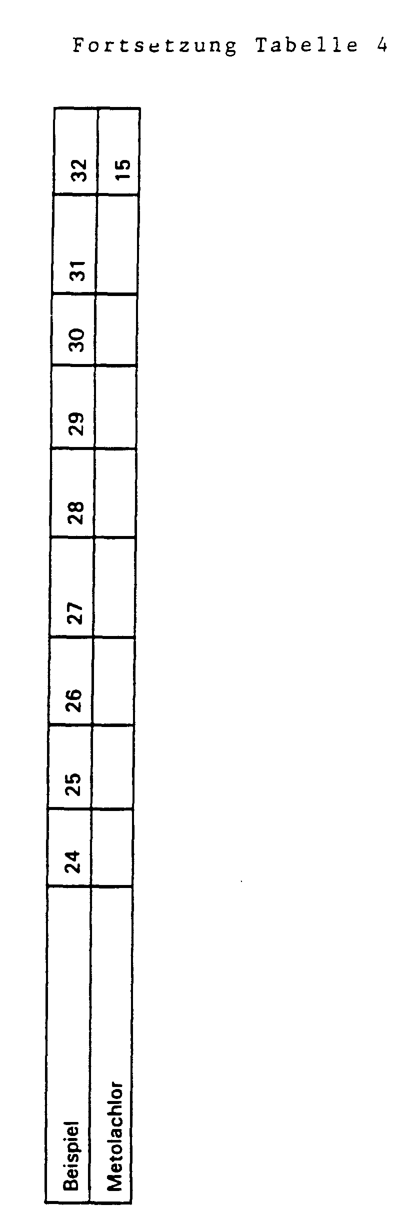

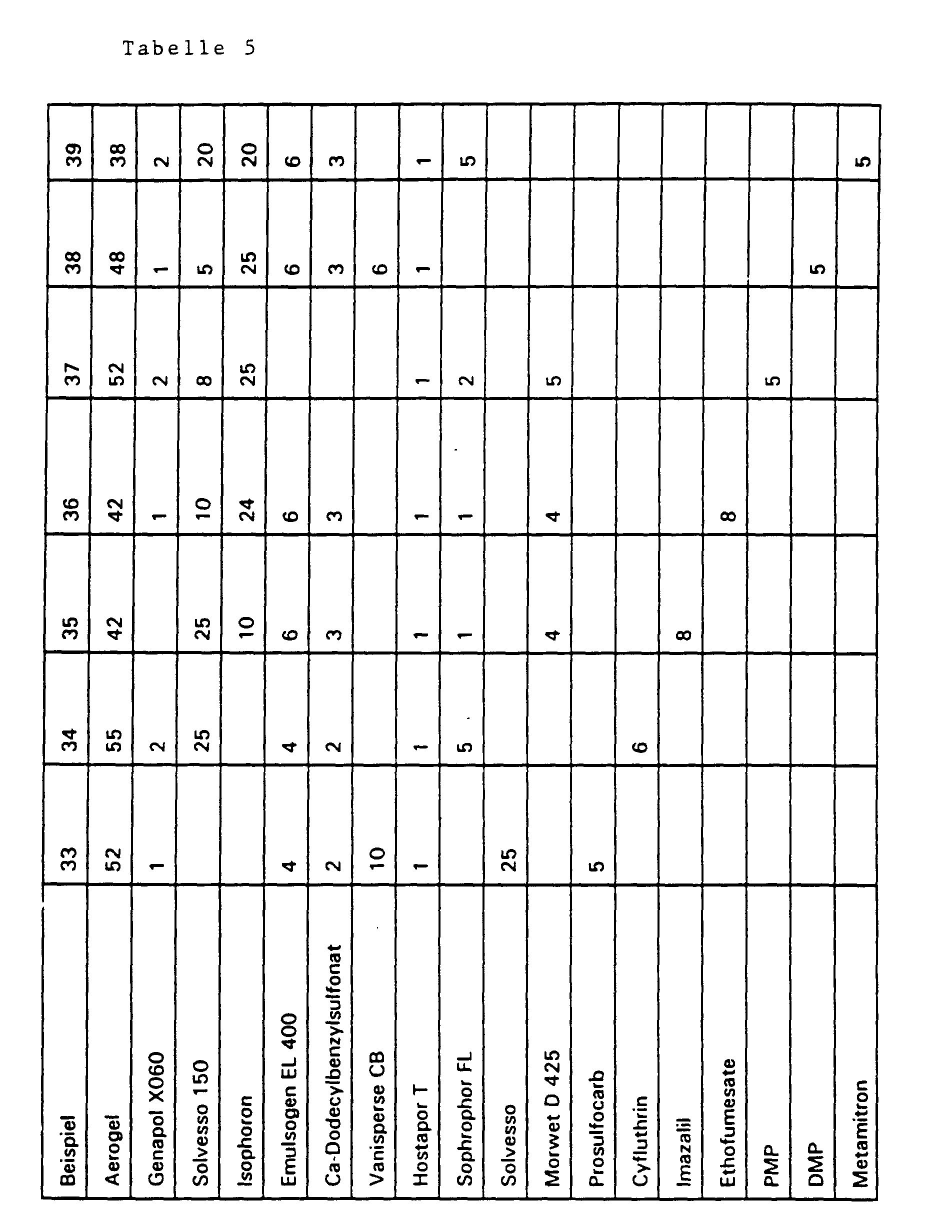

## Patentansprüche

1. Verwendung von durch Silylierung oberflächenmodifizierten Aerogelen als Trägermaterial für aktiv wirksame Substanzen im landwirtschaftlichen und/oder veterinärmedizinischen Bereich.

2. Verwendung von Aerogelen gemäß Anspruch 1 als Trägermaterial für Insekticide, Fungicide, Herbicide, Acaricide, Rhodenticide, Molluscicide, Nematicide, Baktericide und/oder Parasiticide.

3. Verwendung von Aerogelen gemäß Anspruch 1 als Trägermaterial für Viren, Bakterien und/oder Bazillen.

4. Zusammensetzung enthaltend mindestens ein durch Silylierung oberflächenmodifiziertes Aerogel und mindestens eine im landwirtschaftlichen und/oder veterinärmedizinischen Bereich aktiv wirksame Substanz.

5. Zusammensetzung gemäß Anspruch 4, **dadurch gekennzeichnet, daß** die aktiv wirksame Substanz im Trägermedium in flüssiger, gelöster oder suspendierter Form vorliegt.

6. Zusammensetzung gemäß Anspruch 4 oder 5, **dadurch gekennzeichnet, daß** sie mindestens einen Emulgator enthält.

7. Zusammensetzung gemäß mindestens einem der Ansprüche 4 bis 6, **dadurch gekennzeichnet daß** sie mindestens ein Netz- und Dispergiermitel enthält.

## Claims

1. Use of aerogels surface modified by silylation as carrier material for active substances in agriculture and/or veterinary medicine.

2. Use of aerogels according to Claim 1 as carrier material for insecticides, fungicides, herbicides, acaricides, rodenticides, molluscicides, nematicides, bactericides and/or parasiticides.

3. Use of aerogels according to Claim 1 as carrier material for viruses, bacteria and/or bacilli.

4. Composition containing at least one aerogel surface modified by silylation and at least one active substance used in agriculture and/or veterinary medicine.

5. Composition according to Claim 4, **characterised in that** the active substance is present in the carrier medium in liquid, dissolved or suspended form.

6. Composition according to Claim 4 or 5, **characterised in that** it contains at least one emulsifier.

7. Composition according to at least one of Claims 4 to 6, **characterised in that** it contains at least one wetting and dispersing agent.

## Revendications

1. Utilisation d'aérogels modifiés en surface par silylation comme véhicule pour des substances actives dans le domaine agricole et/ou de la médecine vétérinaire.

2. Utilisation d'aérogels selon la revendication 1 comme véhicule pour des insecticides, des fongicides, des herbicides, des acaricides, des rodenticides, des molluequicides, des nématocides, des bactéricides et/ou des parasitocides.

3. Utilisation d'aérogels selon la revendication 1 comme véhicule pour des virus, des bactéries et/ou des bacilles.

4. Composition contenant au moins un aérogel modifié en surface par silylation et au moins une substance efficace active dans le domaine agricole et/ou de la médecine vétérinaire.

5. Composition selon la revendication 4, **caractérisée en ce que** la substance efficace active se présente dans le milieu support sous forme liquide, dissoute ou en suspension.

6. Composition selon la revendication 4 ou 5, **caractérisée en ce qu'**elle contient au moins un agent émulsionnant.

7. Composition selon au moins une des revendications 4 à 6, **caractérisée en ce qu'**elle contient au moins un agent mouillant et dispersant.
